**Europäisches Patentamt**

**European Patent Office**

**Office européen des brevets**

(19)

(11) Publication number: **0 083 338**
**B1**

(12) # EUROPEAN PATENT SPECIFICATION

(45) Date of publication of patent specification: **14.10.87**

(21) Application number: **81902147.8**

(22) Date of filing: **15.07.81**

(86) International application number:
**PCT/US81/00964**

(87) International publication number:
**WO 83/00285 03.02.83 Gazette 83/04**

(51) Int. Cl.⁴: **A 61 K 31/135,**
**A 61 K 31/195, A 61 K 33/14**

(54) COMPOSITION FOR INCREASING BLOOD PRESSURE.

(43) Date of publication of application:
**13.07.83 Bulletin 83/28**

(45) Publication of the grant of the patent:
**14.10.87 Bulletin 87/42**

(84) Designated Contracting States:
**AT CH DE FR GB LI NL SE**

(56) References cited:
**EP-A-0 005 057**
**EP-A-0 005 058**
**FR-A-2 034 543**
**US-A-3 651 237**
**US-A-4 224 343**
**US-A-4 327 112**

**Agressologie 1969, 10(3), pages 199 - 215**
**Science, Vol. 212, issued 1 May 1981,**
**(Washington, D.C., USA) L.A. Conlay,**
**"Thyrosine Increases Blood Pressure in**
**Hypotensive Rats", pp. 559-560.**

(73) Proprietor: **WURTMAN, Richard J.**
**193 Marlborough Street**
**Boston, MA 02116 (US)**

(72) Inventor: **WURTMAN, Richard J.**
**193 Marlborough Street**
**Boston, MA 02116 (US)**

(74) Representative: **Holdcroft, James Gerald, Dr.**
**et al**
**Graham Watt & Co. Riverhead**
**Sevenoaks Kent TN13 2BN (GB)**

(56) References cited:
**Science, Vol. 211, issued 13 February 1981,**
**(Washington, D.C., USA) N.A. Scott, "Tyrosine**
**Administration Decreases Vulnerability to**
**Ventricular Fibrillation in the Normal Canine**
**Heart", pp. 727-729.**

**P.B. Beeson (Ed), "Cecil-Textbook of Medicine",**
**published 1979 by W.B. Saunders Company**
**(Philadelphia, PA), see page 1048.**

Courier Press, Leamington Spa, England.

0 083 338

(58) References cited:
L.S. Goodman (Ed), "The Pharmaceutical Basis
of Therapeutics", 5th Edition, issued 1975 by
Macmillan Publishing Co. (New York, N.Y.),
pages 477-487, 490-492, 494, 495, 500, 501, 503,
506, 507.

New England Journal of Medicine issued 3
October 1974 (Boston, MA, USA) L.I. Goldberg,
Medical Intelligence-Drug Therapy,
"Dopamine-Clinical Uses of an Endogenous
Catecholamine", pp. 707-710.

Chemical Abstracts, Vol. 78, No. 9, issued 5
March 1973 (Columbus, Ohio, USA) Abstract
No. 52807y, H. Achara, "Effect of dopamine on
peripheral vasculature of the rat"

Chemical Abstracts, Vol. 83, No. 23, issued 8
December 1975 (Columbus, Ohio, USA) The
Abstract No. 188282j, T. Furukawa,
"Synergistic effects of ephedrine, tyramine and
norepinephrine on the action of dopanine on
blood"

Chemical Abstracts, Vol. 92, No. 15, issued 14
April 1980, (Columbus, Ohio, USA) Abstract
No. 92:122042k, R.J. Wurtman, "Material and
Composition for reducing blood pressure"

Chemical Abstracts, Vol. 93, No. 7, issued 18
August 1980 (Columbus, Ohio, USA) The
Abstract No. 93:69326, E. Melamed, "Plasma
tyrosine in normal humans: effects of oral
tyrosine and protein - containing meals"

**Description**

This invention relates to a material and composition for increasing blood pressure in a patient having low blood pressure.

It is well known that the neurotransmitter norepinephrine is derived from dihydroxyphenylalanine (DOPA). DOPA is, in turn, produced in neurons by the enzymatic hydroxylation of the amino acid tyrosine. This process is catalyzed by the enzyme tyrosine hydroxylase. The DOPA is decarboxylated to dopamine by the enzyme aromatic L-amino acid decarboxylase (AAAD) and norepinephrine is produced from dopamine in neurons that also contain this reaction chain, the rate-limiting step is the conversion of tyrosine to DOPA. Drugs that act by increasing norepinephrine levels in synapses include the Monoamine Oxidase Inhibitors (which slow the destruction of these neurotransmitters) and the tricyclic antidepressants; these compounds, which are used in treating diseases like depression, are also relatively non-specific—producing many chemical effects besides increasing synaptic norepinephrine levels—and thus have a range of unwanted side effects such as the dangerous increases in blood pressure that occur when people receiving monoamine oxidase inhibitors eat certain foods.

Other diseases appear to be caused by the presence of excessive quantities of norepinephrine within synapses including hypertension and cardiac arrhythmias (too much norepinephrine released from sympathetic neurons). These diseases now usually are treated by drugs that block the interactions of norephinephrine with their post-synaptic receptors. However, these agents all exhibit some non-specific actions as well, as thus cause side-effects.

Prior attempts to increase or decrease the levels of dopamine or norepinephrine by modifying neuronal tyrosine levels had been deemed unsuccessful because the total amounts of these compounds in brains and tissues were not noted to change. It was first observed in Wurtman et al. (Science 185: 183—184, July 12, 1974) that increases in brain DOPA concentrations, which, under the conditions of the experiments varied in proportion to the rates at which dopamine and norepinephrine were being synthesized could be obtained by increasing brain tyrosine concentrations, and that decreases in brain DOPA concentrations could be produced by giving rats treatments that decreased brain tyrosine. An example of a treatment that increased brain tyrosine was the administration of tyrosine itself; an example of a treatment that decreased brain tyrosine was the administration of one of the other neutral amino acids, e.g., leucine, that competes with plasma tyrosine for uptake into the brain. Prior to that disclosure, it had been believed that the rate-limiting enzyme, tyrosine hydroxylase, was so saturated with tyrosine, that increases or decreases in brain tyrosine levels would not affect tyrosine's conversion to DOPA. In neither the above Wurtman et al. article nor a subsequent paper by Gibson and Wurtman (Biochem. Pharmacology, 26:1137—1142, June 1977) was it actually shown that such changes in DOPA accumulation were accompanied by changes in brain dopamine nor norepinephrine levels. Furthermore, in neither was it shown that changing brain tyrosine levels had any effect on the amounts of dopamine nor norepinephrine released into synapses.

The use of tyrosine in the treatment of psychological states of shock, e.g. aggression and fatigue syndromes is disclosed in U.S.—A—3,651,237 (Inventor: Henri Laborit) and its equivalent FR—A—2,034,543. These specifications also describe certain illustrative experiments on rabbits suffering from irreversible hemorrhagic shock but there is no disclosure of the treatment of reversible low blood pressure. Laborit's preferred results on the rabbits were reported using the tyrosine in combination with chlorpromazine.

Further details of Laborit's work are given in Aggressologie 1969, Vol. 10, No. 3, pp 199—215 and on page 203, column 2, first complete paragraph it is indicated that it is essential to use the tyrosine in combination with chlorpromazine.

Other papers authored by Laborit include Aggressologie, 1970, Vol. II, No. 2, pp. 139—151, and Aggressologie, 1969, No. 3, pp. 241—248.

We believe that it would be a mistake to use chlorpromazine along with tyrosine in situations where it is desired to increase blood pressure, inasmuch as this drug blocks certain catecholamine receptors, and can itself lower blood pressure.

It would be highly desirable to provide a means for increasing the amounts of norepinephrine that actually are present within synapses. Such changes in synaptic transmitter levels need not be associated with changes in the total amounts of norepinephrine present in the brain or other tissues, inasmuch as it is now well known that not all of the molecules of the transmitters that are stored in neurons are equally accessible for release into synapses. More specifically, it would be desirable to provide a means for increasing release of norepinephrine in sympathetic neuronal synapses in order to treat conditions to a disease associated with dangerously low blood pressure.

The invention provides use of tyrosine, phenylalanine or a mixture thereof for the manufacture of a medicament for treating low blood pressure (other than orthostatic hypotension) and which is free of chlorpromazine and orally, parenterally or enterally administrable.

The invention also provides use of a composition comprising (a) tyrosine, phenylalanine or a mixture thereof; and (b) a blood pressure increasing agent selected from neosynephrine, calcium chloride, ephedrine, dopamine and norepinephrine, for the manufacture of a medicament for treating low blood pressure (other than orthostatic hypotension) and which is orally, parenterally or enterally administrable.

The invention also provides a pharmaceutical composition free of chlorpromazine and comprising (a)

**0 083 338**

tyrosine, phenylalanine or a mixture thereof and (b) a compound selected from neosynephrine, calcium chloride, ephedrine, dopamine and norepinephrine, the composition being orally, parenterally or enterally administrable.

EP—A—5057 of Massachusetts Institute of Technology discloses the use of tyrosine to decrease rather than increase blood pressure and without being bound by theory it is believed there is a simple explanation for this apparent paradox: it is believed the effect of tyrosine depends upon which nerves are firing in the patient. When the patient has a hypertensive condition, the brain neurons that release norepinephrine are firing so that tyrosine administration enhances brain norepinephrine release which suppresses sympathetic neuron firing, thereby *lowering* blood pressure. When the patient has a hypotensive condition, the brain neurons are not firing. However, the peripheral sympathetic neurons and adrenal medulla cells are very active so that they are receptive to the tyrosine. When tyrosine is administered to the patient, it causes release of norepinephrine into sympathetic neuronal synapses and blood pressure is elevated. In normal tensive cases administered tyrosine does not affect either the brain neurons or the peripheral neurons and blood pressure does not change.

The present invention finds particular application when the patient has a condition apparently characterized by neurons in the sympathetic nervous system that are firing rapidly but are releasing inadequate amounts of norepinephrine. We have found that treatments that increase neuronal tyrosine levels can also cause corresponding increases in the amounts of norepinephrine released into synapses. The tyrosine (or its precursor, phenylalanine) can be administered alone or in admixture with other neutral amino acids with or without drugs, in order to raise tyrosine levels in sympathetic neurons; and thereby to treat low blood pressure associated with deficiency of norepinephrine release by these neurons.

There now follows a description of specific embodiments of the invention which is given by way of example only and not by way of limitation of the invention.

Tyrosine (or phenylalanine) may be administered to a patient either alone or in combination with one or more drugs which cause an increase in blood pressure thereby to increase the level of norepinephrine which is released into sympathetic neuronal synapses. (Serotonin release into brain synapses also can be controlled at the same time by varying the proportion of tryptophan present in the amino acid mixture).

The condition of low blood pressure can arise from any one of a number of causes including conditions wherein the sympathetic nerves are functioning but are unable to release norepinephrine in sympathetic neuron synapses such as in cases of hemorrhaging myocardial infarction, septicemia or shock.

The composition of the amino acid mixture that is utilized depends upon the nature of the illness in the patient that is to be treated. When there is need to increase norepinephrine release in sympathetic neuronal synapses without increasing that of brain serotonin, tyrosine (and/or phenylalanine) is administered, with or without other amino acids not including serotonin's percursor, tryptophan, in doses ranging between 5 mg/kg and 200 mg/kg. In some situations, phenylalanine can be used as a substitute for tyrosine, inasmuch as much of this amino acid is converted to tyrosine in the liver.

The amount of tyrosine or phenylalanine administered is between 2 and 200 mg/kg body weight/day preferably between 10 and 100 mg/kg body weight/day in order to obtain tyrosine or phenylalanine blood plasma concentrations between 15 and 100 micrograms/ml, preferably between 20 and 40 micrograms/ml, thereby to obtain effective increases in blood pressure. When the amino acid is administered in a liquid carrier such as saline or sugar solution, the concentration of tyrosine or phenylalanine should be between about 5 mg/ml and 100 mg/ml. The tyrosine can be administered orally, parenterally or enterally.

The tyrosine or phenylalanine can be administered as free amino acids, esters, salts, natural or synthetic polymers, or as constituents of foods. The route of administration can be oral or parenteral, e.g., intravenous. Tyrosine and/or phenylalanine also can be administered alone or with a drug known to increase blood pressure including neosynephrine, calcium chloride, ephedrine, dopamine or norepinephrine.

Example

This example illustrates that an animal having low blood pressure induced by hemorrhaging the animal can have its blood pressure increased into normal range by administering tyrosine to the animal.

Male Sprague-Dawley rats (Charles River Breeding Laboratories, Wilmington, MA) weighing 350—450 g were housed individually, given *ad libitum* access to tap water and a stock diet, and maintained under light (300 microwatts/cm$^2$; Vita-Lite, Duro-Test Corp., North Bergen, NJ) between 7 AM and 7 PM daily.

Tyrosine (methyl ester) was dissolved in saline and buffered to pH 7.4 with sodium hydroxide.

A group of 12 rats were hemorrhaged, via the carotid artery, to reduce their calculated blood volume by 15%; blood pressure was reduced by 4000±667 Pa (30±5 mm mercury). After about 15 minutes, the animals then were administered intravascularly either about 0.2 ml saline or about 0.2 of a tyrosine solution in saline comprising 100 mg tyrosine/ml saline. The tyrosine dosage corresponded to 100 mg/kg animal body weight. The effects of saline and of tyrosine on blood pressure, 5, 15 or 25 minutes following administration, is shown in Table I.

TABLE

| Treatment | Time (minutes) | | |
|---|---|---|---|
| | 5 | 15 | 25 |
| | (Rise in Blood Pressure—Pa—(mmHg)) | | |
| Saline | 2400±1333 (18±10) | 1333± 933 (10± 7) | 1467± 933 (11± 7) |
| Tyrosine | 8267±2667 (62±20) | 8533±3200 (64±24) | 5067±2000 (38±15) |

As shown in the Table tyrosine, but not saline, not only restores blood pressure but actually raises it above normal.

In order to determine whether tyrosine can prevent the development of hemorrhage-induced low blood pressure, groups of 4 rats were intravascularly administered 100 mg/kg body weight tyrosine (100 mg/ml, in saline) comparable volume of saline alone. Five minutes after the tyrosine administration both groups of rats were bled by 10%, 15% or 20% of their total blood volume. Systolic blood pressures in the animals given saline fell 4000±667 Pa (30±5 mmHg), 4000±933 Pa (30±7 mmHg) or 6667±1333 Pa (50±10 mmHg) respectively. Systolic blood pressures in animals given the tyrosine fell by only 2400±667 Pa (18±5 mmHg), 2267±533 Pa (17±4 mmHg) or 3867±1067 Pa (29±8 mmHg), respectively. Hence, tyrosine has utility in the treatment and the prophylaxis or hemorrhage-induced low blood pressure.

**Claims for the Contracting States: CH, DE, FR, GB, LI, NL, SE**

1. Use of tyrosine, phenylalanine or a mixture thereof for the manufacture of a medicament for treating low blood pressure (other than orthostatic hypotension) and which is free of chlorpromazine and orally, parenterally or enterally administrable.

2. Use of a composition comprising (a) tyrosine, phenylalanine or a mixture thereof; and (b) a blood pressure increasing agent selected from neosynephrine, calcium chloride, ephedrine, dopamine and norepinephrine, for the manufacture of a medicament for treating low blood pressure (other than orthostatic hypotension) and which is orally, parenterally or enterally administrable.

3. A pharmaceutical composition free of chlorpromazine and comprising (a) tyrosine, phenylalanine or a mixture thereof and (b) a compound selected from neosynephrine, calcium chloride, ephedrine, dopamine and norepinephrine, the composition being orally, parenterally or enterally administrable.

4. A composition according to claim 3, for use in treating low blood pressure (other than orthostatic hypotension) in e.g. humans.

**Claims for the Contracting State: AT**

1. Use of tyrosine, phenylalanine or a mixture thereof for the manufacture of a medicament for treating low blood pressure (other than orthostatic hypotension) and which is free of chlorpromazine and orally, parenterally or enterally administrable.

2. Use of a composition comprising (a) tyrosine, phenylalanine or a mixture thereof; and (b) a blood pressure increasing agent selected from neosynephrine, calcium chloride, ephedrine, dopamine and norepinephrine, for the manufacture of a medicament for treating low blood pressure (other than orthostatic hypotension) and which is orally, parenterally or enterally administrable.

3. A method of manufacturing a pharmaceutical composition which is free of chlorpromazine and is orally, parenterally or enterally administrable, comprising the steps of mixing (a) tyrosine, phenylalanine or a mixture thereof with (b) a compound selected from neosynephrine, calcium chloride, ephedrine, dopamine and norepinephrine.

**Patentansprüche für die Vertragsstaaten: CH, DE, FR, GB, LI, NL, SE**

1. Verwendung von Tyrosin, Phenylalanin oder eines Gemisches dieser Substanzen zur Herstellung eines Medikaments zum Behandeln von Niedrigblutdruk (außer orthostatischer Hypotension), das frei von Chlorpromazin und oral, parenteral oder enteral verabreichungsfähig ist.

2. Verwendung einer Zusammensetzung, bestehend aus a) Tyrosin, Phenylalanin oder einem Gemisch dieser Substanzen und b) einem aus der Gruppe Neosynephrin, Kalziumchlorid Ephedrin, Dopamin und Norepinephrin ausgewählten Blutdruckerhöhungsmittel zur Herstellung eines Medikaments zur Behandlung von Niedrigblutdruck (außer orthostatischer Hypotension), das oral, parenteral oder enteral verabreichungsfähig ist.

3. Chlorpromazinfreie pharmazeutische Zusammensetzung, bestehend aus a) Tyrosin, Phenylalanin oder einem Gemisch dieser Substanzen und b) einem aus der Gruppe Neosynephrin, Kalziumchlorid, Ephedrin, Dopamin und Norepinephrin ausgewählten Compound, wobei die Zusammensetzung oral, parenteral oder enteral verabreichungsfähig ist.

4. Zusammensetzung nach Anspruch 3, zur Verwendung bei der Behandlung von Niedrigblutdruck (außer orthostatischer Hypotension) an z.B. Menschen.

**Patentansprüche für den Vertragsstaat: AT**

1. Verwendung von Tyrosin, Phenylalanin oder eines Gemisches dieser Substanzen zur Herstellung eines Medikaments zum Behandeln von Niedrigblutdruck (außer orthostatischer Hypotension), das frei von Chlorpromazin und oral, parenteral oder enteral verabreichungsfähig ist.

2. Verwendung einer Zusammensetzung, bestehend aus a) Tyrosin, Phenylalanin oder einem Gemisch dieser Substanzen und b) einem aus der Gruppe Neosynephrin, Kalziumchlorid, Ephedrin, Dopamin und Norepinephrin ausgewählten Blutdruckerhöhungsmittel zur Herstellung eines Medikaments zur Behandlung von Niedrigblutdruck (außer orthostatischer Hypotension), das oral, parenteral oder enteral verabreichungsfähig ist.

3. Verfahren zur Herstellung einer pharmazeutischen Zusammensetzung, die frei von Chlorpromazin und oral, parenteral oder enteral verabreichungsfähig ist, bei dem a) Tyrosin, Phenylalanin oder ein Gemisch dieser Substanzen mit b) einem aus der Gruppe Neosynephrin, Kalziumchlorid, Ephedrin, Dopamin und Norepinephrine ausgewählten Compound gemischt werden.

**Revendications pour les Etats Contractants: CH, DE, FR, GB, LI, NL, SE**

1. Utilisation de tyrosine, de phénylalanine ou d'un mélange de ces substances pour la fabrication d'un médicament qui est utilisable dans le traitement de basses tensions artérielles (autres que l'hypotension orthostatique), qui est exempt de chlorpromazine et qui peut être administré par voie buccale, parentérale ou intestinale.

2. Utilisation d'une composition comprenant (a) de la tyrosine, de la phénylalanine ou un mélange de ces substances et (b) un agent hypertensif choisi parmi la néosynéphrine, le chlorure de calcium, l'éphédrine, la dopamine et la norépinéphrine, pour la fabrication d'un médicament qui est utilisable dans le traitement de basses tensions artérielles (autres que l'hypotension orthostatique) et qui peut être administré par voie buccale, parentérale ou intestinale.

3. Composition pharmaceutique exempte de chlorpromazine et comprenant (a) de la tyrosine, de la phénylalanine ou un mélange de ces substances et (b) un composé choisi parmi la néosynéphrine, le chlorure de calcium, l'éphédrine, la dopamine et la norépinéphrine, cette composition pouvant être administrée par voie buccale, parentérale ou intestinale.

4. Composition selon la revendication 3, utilisable dans le traitement de basses tensions artérielles (autres que l'hypotension orthostatique), par exemple chez l'homme.

**Revendications pour l'Etat Contractant: AT**

1. Utilisation de tyrosine, de phénylalanine ou d'un mélange de ces substances pour la fabrication d'un médicament qui est utilisable dans le traitement de basses tensions artérielles (autres que l'hypotension orthostatique), qui est exempt de chlorpromazine et qui peut être administré par voie buccale, parentérale ou intestinale.

2. Utilisation d'une composition comprenant (a) de la tyrosine, de la phénylalanine ou un mélange de ces substances et (b) un agent hypertensif choisi parmi la néosynéphrine, le chlorure de calcium, l'éphédrine, la dopamine et la norépinéphrine, pour la fabrication d'un médicament qui est utilisable dans le traitement de basses tensions artérielles (autres que l'hypotension orthostatique) et qui peut être administré par voie buccale, parentérale ou intestinale.

3. Procédé de préparation d'une composition pharmaceutique qui est exempte de chlorpromazine et qui peut être administrée par voie buccale, parentérale ou intestinale, comprenant les phases consistant à mélanger (a) de la tyrosine, de la phénylalanine ou un mélange de ces substances avec (b) un composé choisi parmi la néosynéphrine, le chlorure de calcium, l'éphédrine, la dopamine et la norépinéphrine.